# EUROPÄISCHE PATENTSCHRIFT

(11) **EP 1 424 985 B1**
(45) Veröffentlichungstag und Bekanntmachung des Hinweises auf die Patenterteilung: **13.02.2008**
(21) Anmeldenummer: 02779265.4
(22) Anmeldetag: 17.08.2002
(51) Int. Cl.: A61K 31/255, A61P 3/04

(54) **VERWENDUNG VON DERIVATEN VON C2-SUBSTITUIERTEN INDAN-1-OL-SYSTEMEN ZUR HERSTELLUNG VON MEDIKAMENTEN ZUR PROPHYLAXE ODER BEHANDLUNG VON OBESITAS**
USE OF DERIVATIVES OF C2-SUBSTITUTED INDANE-1-OL SYSTEMS FOR PRODUCING MEDICAMENTS FOR THE PROPHYLAXIS OR THE TREATMENT OF OBESITY
UTILISATION DE DERIVES DE SYSTEMES D'INDANE-1-OL C2-SUBSTITUES POUR PRODUIRE DES MEDICAMENTS PERMETTANT DE PREVENIR OU TRAITER L'OBESITE

(30) Priorität: 31.08.2001 DE 10142660
(43) Veröffentlichungstag der Anmeldung: 09.06.2004
(73) Patentinhaber: Sanofi-Aventis Deutschland GmbH, 65929 Frankfurt am Main (DE)
(72) Erfinder: JAEHNE, Gerhard, 65929 Frankfurt (DE); KRONE, Volker, 65719 Hofheim (DE); BICKEL, Martin, 61348 Bad Homburg (DE); GOSSEL, Matthias, 65719 Hofheim (DE)
(86) Internationale Anmeldenummer: PCT/EP2002/009199
(87) Internationale Veröffentlichungsnummer: WO 2003/020199

(56) Entgegenhaltungen:
- EP-A- 0 009 554
- WO-A-01/02373
- WO-A-01/62746
- WO-A-97/20806

## Beschreibung

Verwendung von Derivaten von C2-substituierten Indan-1-ol-Systemen zur Herstellung von Medikamenten zur Prophylaxe oder Behandlung von Obesitas.

Die Erfindung betrifft die Verwendung von Derivaten von C2-substituierten Indan-1-ol-Systemen sowie derer physiologisch verträgliche Salze zur Herstellung von Medikamenten zur Prophylaxe oder Behandlung von Obesitas.

In EP 0009554 werden Indan-1-on- und -1-ol-Derivate als Herbizide und Analgetika offenbart.

In WO 97/20806 werden cyclopentyl-substituierte Indan-1-on-Derivate mit u.a. entzündungshemmender Wirkung offenbart.

In WO 01/023373 wird ein mit Indanol substituiertes Dihydrobenzodiazepin zur Behandlung von Dyslipidämie offenbart.

Der Erfindung lag die Aufgabe zugrunde, Verbindungen zur Verfügung zu stellen, die zur Gewichtsreduktion von Säugetieren verwendet werden können und die insbesonders eine therapeutisch verwertbare anorektische Wirkung entfalten.

Die Erfindung betrifft daher die Verwendung der Verbindungen der Formel I, 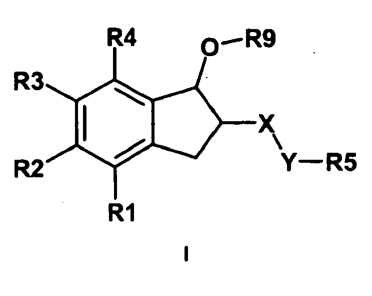 worin bedeuten
- R1, R2, R3, R4: unabhängig voneinander H, F, Cl, Br, J, CN, N₃, NO₂, OH, O(C₁-C₈)-Alkyl, O(C₃-C₈)-Cycloalkyl, O-CH₂-phenyl, O-phenyl, O-CO-(C₁-C₈)-Alkyl, O-C0-(C₃-C₈)-Cycloalkyl, S(O)₀₋₂(C₁-C₈)-Alkyl, S(O)₀₋₂(C₃-C₈)-Cycloalkyl, NH₂, NH-(C₁-C₈)-Alkyl, NH-(C₃-C₈)-Cycloalkyl, N[(C₁-C₈)-Alkyl]₂, N[(C₃-C₈)-Cycloalkyl]₂, NH-CO-(C₁-C₈)-Alkyl, NH-CO-(C₃-C₈)-Cycloalkyl; SO₃H, SO₂-NH₂, SO₂-NH-(C₁-C₈)-Alkyl, SO₂-NH-(C₃-C₈)-Cycloalkyl, NH-SO₂-NH₂, NH-SO₂-(C₁-C₈)-Alkyl, NH-SO₂-(C₃-C₈)-Cycloalkyl, O-CH₂-COOH, O-CH₂-CO-O(C₁-C₈)-Alkyl, O-CH₂-COOH, O-CH₂-CO-O(C₁-C₈)-Alkyl, COOH, CO-O(C₁-C₈)-Alkyl, CO-O-(C₃-C₈)-Cycloalkyl, CO-NH₂, CO-NH(C₁-C₈)-Alkyl, CO-N[(C₁-C₈)-Alkyl]₂, (C₁-C₈)-Alkyl, (C₃-C₈)-Cycloalkyl, (C₂-C₈)-Alkenyl, (C₂-C₈)-Alkinyl, wobei in den Alkyl-, Alkenyl- und Alkinylgruppen jeweils ein bis zu sieben Wasserstoffatome durch Fluor ersetzt sein können, oder ein Wasserstoff durch OH, OC(O)CH₃, O-CH₂-Ph, NH₂, NH-CO-CH₃ oder N(COOCH₂Ph)₂ ersetzt sein kann; Phenyl, 1- oder 2-Naphthyl,
5-Tetrazolyl, 1-[(C₁-C₆)-Alkyl]-5-Tetrazolyl, 2-[(C₁-C₆)-Alkyl]-5-Tetrazolyl,
1-Imidazolyl,
1-oder 4-[1,2,4]Triazolyl,
2- oder 3-Thienyl,
2- oder 3-Furyl,
2-, 3- oder 4-Pyridyl,
2-, 4- oder 5-Oxazolyl,
3-, 4- oder 5-lsoxazolyl,
2-, 4- oder 5-Thiazolyl,
3-, 4- oder 5-Isothiazolyl
wobei der Arylrest oder Heterocyclus bis zu zweifach mit F, Cl, Br, CN, OH, (C₁-C₄)-Alkyl, CF₃, O-(C₁-C₄)-Alkyl, S(O)₀₋₂(C₁-C₆)-Alkyl, NH₂, NH-SO₂-(C₁-C₄)-Alkyl, COOH, CO-O-(C₁-C₄)-Alkyl, CO-NH₂ substituiert sein kann und in den Alkylgruppen ein bis sieben Wasserstoffatome durch Fluor ersetzt sein können;
- oder R2 und R3: bilden gemeinsam die Gruppe -O-CH₂-O-
- X: S, SO, SO₂;
- Y: (CH₂)ₚ, wobei p gleich 0, 1, 2 oder 3 sein kann;
- R5: CF₃, (C₁-C₁₈)-Alkyl, (C₃-C₈)-Cycloalkyl, wobei in den Alkylgruppen ein bis sieben Wasserstoffatome durch Fluor ersetzt sein können;
(CH₂)ᵣCOR6, wobei r = 1-6 sein kann und R6 gleich OH, O-(C₁-C₆)-Alkyl oder NH₂ sein kann;
CH₂-CH(NHR7)-COR8, wobei R7 gleich H oder C(O)-(C₁-C₄)-Alkyl und R8 gleich OH, 0-(C₁-C₆)-Alkyl oder NH₂ sein kann;
Phenyl, 1- oder 2-Naphthyl, Biphenyl oder ein Heterocyclischer Rest, wobei die Ringe oder Ringsysteme bis zu zweifach substituiert sein können mit F, Cl, Br, J, CN, OH, O(C₁-C₈)-Alkyl, O(C₃-C₈)-Cycloalkyl, O-CO-(C₁-C₈)-Alkyl, O-CO-(C₃-C₈)-Cycloalkyl, S(O)₀₋₂(C₁-C₈)-Alkyl, S(O)₀₋₂(C₃-C₈)-Cycloalkyl, NH₂, NH-(C₁-C₈)-Alkyl, NH-(C₃-C₈)-Cycloalkyl, N[(C₁-C₈)-Alkyl]₂, N[(C₃-C₈)-Cycloalkyl]₂, NH-CO-(C₂-C₈)-Alkyl, NH-CO-(C₃-C₈)-Cycloalkyl; SO₃H, SO₂-NH₂, SO₂-NH-(C₁-C₈)-Alkyl, SO₂-NH-(C₃-C₈)-Cycloalkyl; NH=SO₂-NH₂, NH-SO₂-(C₁-C₈)-Alkyl, NH-SO₂-(C₃-C₈)-Cycloalkyl, O-CH₂-COOH, O-CH₂-CO-O(C₁-C₈)-Alkyl, COOH, CO-O(C₁-C₈)-Alkyl, CO-O-(C₃-C₈)-Cycloalkyl, CO-NH₂, CO-NH(C₁-C₈)-Alkyl, CO-N[(C₁-C₈)-Alkyl]₂, (C₁-C₈)-Alkyl, (C₃-C₈)-Cycloalkyl, wobei in den Alkylgruppen jeweils ein bis zu sieben Wasserstoffatome durch Fluor ersetzt sein können;
- R9: (C₁-C₁₂)-Alkyl, (C₃-C₈)-Cycloalkyl, wobei in den Alkylresten ein bis sieben Wasserstoffatome durch Fluor ersetzt sein können;
CO-O(C₁-C₆)-Alkyl, CO-O(C₃-C₈)-Cycloalkyl, C(O)-(C₁-C₈)-Alkyl, C(O)-(C₃-C₈)-Cycloalkyl, C(O)-Phenyl, C(O)-CH(NHR12)-(C₁-C₈)-Alkyl, Phenyl, 1-oder 2-Naphthyl, Biphenyl, 2-, 3- oder 4-Pyridyl, wobei die Aryl- oder Heteroarylreste bis zu zweifach mit F, Cl, Br, CN, OH, (C₁-C₄)-Alkyl, CF₃, O-(C₁-C₄)-Alkyl, S(O)₀₋₂(C₁-C₆)-Alkyl, NH₂, NH-SO₂-(C₁-C₄)-Alkyl, -CH₂-COOH, O-CH₂-CO-O(C₁-C₈)-Alkyl, COOH, CO-O-(C₁-C₄)-Alkyl, CO-NH₂ substituiert sein können; (CH₂)-R10;
(CH₂)ₛ-R11, wobei s = 2 oder 3 sein kann;
- R10: (C₁-C₁₂)-Alkyl, (C₃-C₈)-Cycloalkyl, wobei in den Alkylresten ein bis sieben Wasserstoffatome durch Fluor ersetzt sein können; COOH, CONH₂, CO-O(C₁-C₆)-Alkyl, CO-O(C₃-C₈)-Cycloalkyl; Phenyl, 1-oder 2-Naphthyl, Biphenyl, 2-, 3- oder 4-Pyridyl, 2- oder 3-Furyl oder 2- oder3-Thienyl sein kann, wobei die Aryl- oder Heteroarylreste bis zu zweifach mit F, Cl, Br, CN, OH, (C₁-C₄)-Alkyl, CF₃, O-(C₁-C₄)-Alkyl, S(O)₀₋₂(C₁-C₆)-Alkyl, NH₂, NH-SO₂-(C₁-C₄)-Alkyl, O-CH₂-COOH, O-CH₂-CO-O(C₁-C₈)-Alkyl, COOH, CO-O-(C₁-C₄)-Alkyl, CO-NH₂ substituiert sein können;
- R11: (C₁-C₁₂)-Alkyl, (C₃-C₈)-Cycloalkyl, wobei in den Alkylresten ein bis sieben Wasserstoffatome durch Fluor ersetzt sein können; COOH, CONH₂, CO-O(C₁-C₆)-Alkyl, CO-O(C₃-C₈)-Cycloalkyl; Phenyl, 1-oder 2-Naphthyl, Biphenyl, 2-, 3- oder 4-Pyridyl, 2- oder 3-Furyl, 2- oder 3-Thienyl oder 1-Imidazolyl, wobei die Aryl- oder Heteroarylreste bis zu zweifach mit F, Cl, Br, CN, OH, (C₁-C₄)-Alkyl, CF₃, O-(C₁-C₄)-Alkyl, S(O)₀₋₂(C₁-C₆)-Alkyl, NH₂, NH-SO₂-(C₁-C₄)-Alkyl, O-CH₂-COOH, O-CH₂-CO-O(C₁-C₈)-Alkyl, COOH, CO-O-(C₁-C₄)-Alkyl, CO-NH₂ substituiert sein können;
- R12: H, C(O)-(C₁-C₆)-Alkyl;
sowie derer physiologisch verträgliche Salze zur Herstellung eines Medikaments zur Gewichtsreduktion von Säugetieren.

Die Erfindung bezieht sich auf Verwendung der Verbindungen der Formel 1, in Form ihrer Racemate, racemischen Mischungen und reinen Enantiomere sowie auf ihre Diastereomere und Mischungen davon.
Die Alkyl-, Alkenyl- und Alkinylreste in den Substituenten R1, R2, R3, R4, R5, R6, R7, R8, R9, R10, R11 und R12 können sowohl geradkettig wie verzweigt sein.

Unter Heterocyclus bzw. Heterocyclischer Rest werden Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der Heterocyclische Rest mit Benzolkernen kondensiert ist. Bevorzugte Hetercyclen bzw. Heterocyclische Reste sind:
Heteroaryl wie
Benzimidazolyl,
1-[(C₁-C₆)-Alkyl]-Benzimidazolyl,
Imidazolyl,
2- oder 3-Thienyl,
2- oder 3-Furyl,
Benzoxazolyl,
Benzthiazoyl,
2-, 3- oder 4-Pyridyl,
Pyrimidinyl,
4-, 5- oder 6-Pyridazin-2H-3-on-yl,
4-, 5- oder 6-Pyridazin-2-(C₁-C₈)-Alkyl-2H-3-on-yl,
4-, 5- oder 6-Pyridazin-2-benzyl-2H-3-on-yl,
3- oder 4-Pyridazinyl,
2-, 3-, 4- oder 8-Chinolinyl,
1-, 3- oder 4-Isochinolinyl,
1-Phthalazinyl,
3- oder 4-Cinnolinyl,
2- oder 4-Chinazolinyl,
2-Pyrazinyl,
2-Chinoxalinyl,
2-, 4- oder 5-oxazolyl,
3-, 4- oder 5-lsoxazolyl,
2-, 4- oder 5-Thiazolyl,
3-, 4- oder 5-Isothiazolyl,
1-[(C₁-C₆)-Alkyl]-2-, 4- oder 5-lmidazolyl,
3-, 4- oder 5-Pyrazolyl,
1-[(C₁-C₆)-Alkyl]-3-, 4- oder 5-Pyrazolyl,
1- oder 4-[1,2,4]Triazolyl,
4- oder 5-[1,2,3]Triazolyl,
1-[(C₁-C₆)-Alkyl]-4- oder 5-[1.2.3]Triazolyl,
3-, 4- oder 7-Indolyl,
N-[(C₁-C₆)-Alkyl]-3-, 4- oder 7-Indolyl
2-[(C₁-C₆)-Alkyl]-3(2H)-Indazolyl,
1-[(C₁-C₆)-Alkyl]-3(1H)-Indazolyl,
5-Tetrazolyl,
1-[(C₁-C₆)-Alkyl]-1H-Tetrazolyl,
2-[(C₁-C₆)-Alkyl]-2H-Tetrazolyl.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der Verbindungen der Formel I sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isäthion-, Milch-, Lactobion-, Malein-, Apfel-, Methansulfon-, Bemstein-, p-Toluolsulfon-, Wein- und Trifluoressigsäure ferner L-Ascorbinsäure, Salizylsäure, 1,2-Benzisothiazol-3(2H)-on und 6-Methyl-1,2,3-oxathiazin-4(3H)-on-2,2-dioxid. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Die Verbindungen der Formel I können auch in verschiedenen polymorphen Formen vorliegen, z.B. als amorphe und kristalline polymorphe Formen. Alle polymorphen Formen der Verbindungen der Formel I gehören in den Rahmen der Erfindung.

Nachfolgend beziehen sich alle Verweise auf "Verbindung(en) gemäß Formel (I)" auf Verbindung(en) der Formel (I) wie vorstehend beschrieben, sowie ihre Salze und Solvate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Benzothiazepin-lons. Zur Prophylaxe oder Therapie der oben genannten Zustände können die Verbindungen gemäß Formel (1) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Verbindungen der der allgemeinen Formel I, können gemäß den folgenden Reaktionsschemata hergestellt werden:

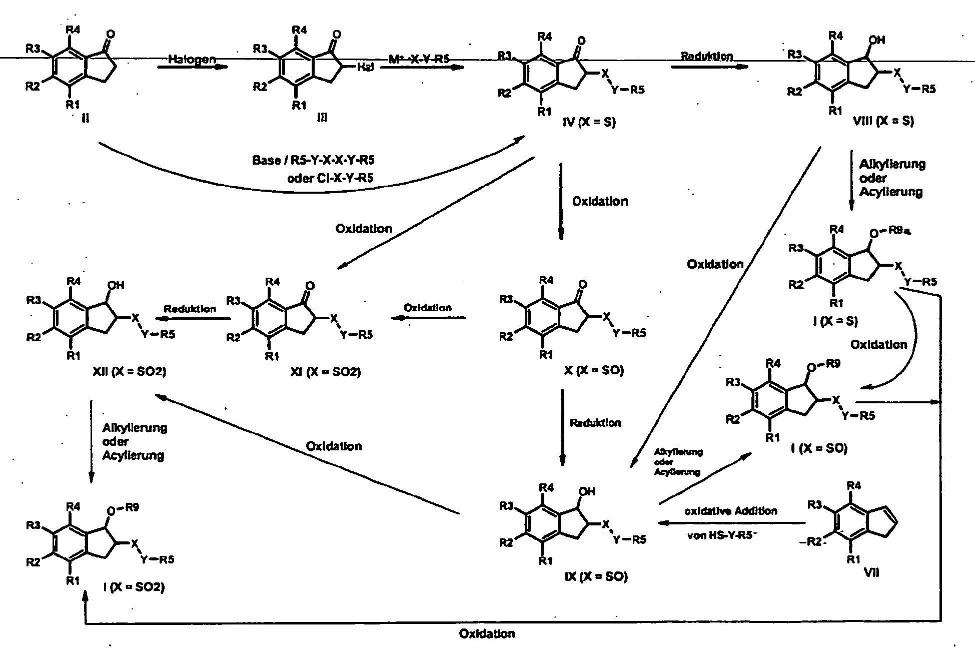

Dazu werden Verbindungen der allgemeinen Formel II, 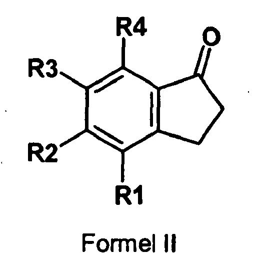 worin R1, R2, R3 und R4 die angegebene Bedeutung besitzen, mit einem Halogen wie z. B. Brom oder Chlor in eine Verbindung der Formel III überführt.
Die Verbindungen der Formel III werden weiter mit Metallsalzen von Thiolen der allgemeinen Formel H-X-Y-R5, wobei X gleich Schwefel ist und Y und R5 die angegebenen Bedeutungen besitzen, in Verbindungen der Formel IV mit X = S überführt. Diese Metallsalze können als solche eingesetzt werden oder in Lösung *in situ* aus dem Thiol und einer Base wie z. B. wässrigem Natriumhydroxid erzeugt werden.
Andererseits kann man Verbindungen der Formel IV mit X = S durch Umsatz von Verbindungen der Formel II mit einer Base wie z.B. Lithiumdiisopropylamid in z.B. Tetrahydrofuran und einem Disulfid der allgemeinen Formel R5-Y-X-X-Y-R5, worin R5 und Y die angegebenen Bedeutungen besitzen und X = S ist, gewinnen; alternativ kann statt des Disufids auch ein Sulfenylchlorid der allgemeinen Formel Cl-X-Y-R5,
wobei X = S ist und Y und R5 die angegebenen Bedeutungen haben, eingesetzt werden (siehe z.B. D. Seebach et al.; Chem. Ber. 109, 1601-1616 (1976)).

Verbindungen der Formel X, worin X = SO ist, können z.B. durch selektive Oxidation der Verbindung der Formel IV, worin X = S ist, mit einem Äquivalent Peroxytrifluoressigsäure (C. G. Venier et al.; J. Org. Chem. 47, 3773 (1982)) hergestellt werden. Die Herstellung der Sulfoxide aus den Sulfiden kann auch mittels Mangandioxid oder Chromsäure erfolgen (D. Edwards et al.; J. Chem. Soc. 1954, 3272). Für diese Oxidation ist weiterhin auch Wasserstoffperoxid in Essigsäureanhydrid geeignet (A. V. Sviridova et al.; J. Org. Chem (Russ), English Transl.; 7, 2577 (1971).

Verbindungen der Formel XI, worin X = SO₂ ist, können durch Oxidation mit z.B. 2KHSO₅ x KHSO₄ x K₂SO₄ (Oxone) entweder aus Verbindungen der Formel IV, worin X = S ist, oder aus Verbindungen der Formel X, worin X = SO ist, gewonnen werden (siehe z.B. M. Hudlicky, Oxidations in Organic Chemistry, ACS Monograph 186, American Chemical Society, Washington, DC, 1990).

Verbindungen der Formel X, worin X = SO oder der Formel XI, worin X = SO₂ und Y = eine Bindung (= (CH₂)ₘ mit m = 0) ist, können auch alternativ nach dem folgenden Schema (gezeigt für die Darstellung der Arylsulfoxide (H. J. Monteiro et al.; Tetrahedron Letters 11, 921-924 (1975) und Arylsulfone (A. K. Maiti et al.; Tetrahedron 50, 10483-10490 (1994)) hergestellt werden:

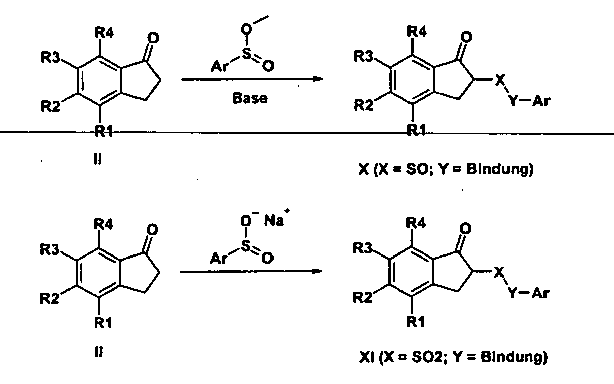

Durch Reduktion mit z.B. Lithiumaluminiumhydrid oder Natriumborhydrid können die Ketone der Formel IV, worin X = S ist, in die Alkohole der Formel VIII, worin X = S ist, überführt werden. In ähnlicher Weise lassen sich die Verbindungen der Formel X, worin X = SO ist, in die Verbindungen der Formel IX, worin X = SO ist, umwandeln und auch die Verbindungen der Formel XI, worin X = SO₂ ist, in solche Verbindungen XII, worin X = SO2 ist. Ferner lassen sich Verbindungen der Formel IX, worin X = SO ist, durch Oxidation in Verbindungen der Formel XII, worin X = SO₂ ist, umwandeln. Durch Oxidation lassen sich auch Verbindungen der Formel IX, worin X =SO ist, aus Verbindungen der Formel VIII, worin X = S ist, herstellen.
Indene der Formel VII lassen sich durch oxidative Addition von Verbindungen der Formel HX-Y-R5, worin X = S ist, in Verbindungen der Formel IX, worin X = SO ist, umwandeln (siehe z.B. J. F. Ford et al. (Tetrahedron 4, 325-336 (1958)). Verbindungen der Formel I, worin X = S ist, lassen sich durch Alkylierung oder Acylierung aus Verbindungen der Formel VIII, worin X = S ist, gewinnen. Verbindungen der Formel I, worin X = SO ist, lassen sich entweder durch Oxidation aus Verbindungen der Formel I, worin X = S ist, herstellen oder durch Alkylierung oder Acylierung aus Verbindungen der Formel IX, worin X = SO, ist. Verbindungen der Formel 1, worin X = SO₂ ist, lassen sich durch Oxidation von Verbindungen der Formel, worin X = S oder SO ist, herstellen oder durch Alkylierung oder Acylierung von Verbindungen der Formel XII, worin X = SO₂ ist.

Verbindungen der Formel, worin X = S ist, und die Reste R1 bis R9 die angegebenen Bedeutungen haben, lassen sich auch über die Addition von Verbindungen des Typs R5-Y-X-H, worin X = S ist, an Indene der Formel VII über die Chlorverbindungen der Formel XIII und nachfolgendem Umsatz mit Verbindungen der Formel H-O-R9 oder M-O-R9, wobei M für z.B. ein Alkalimetall steht, gewinnen:

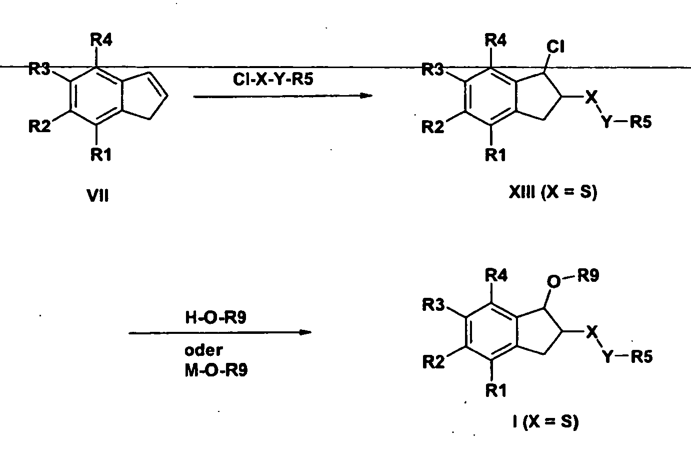

Verbindungen der Formel I, worin X = SO₂ ist, können auch gewonnen werden, indem Epoxide der Formel XIV mit Sulfinsäuresalzen der Formel R5-Y-X-Na, worin X = SO₂ ist, zu Verbindungen der Formel XII, worin X = SO₂ ist, umgesetzt und nachfolgend einer Alkylierung oder Acylierung zu Verbindungen der Formel I, worin X = SO₂ ist, unterworfen werden:

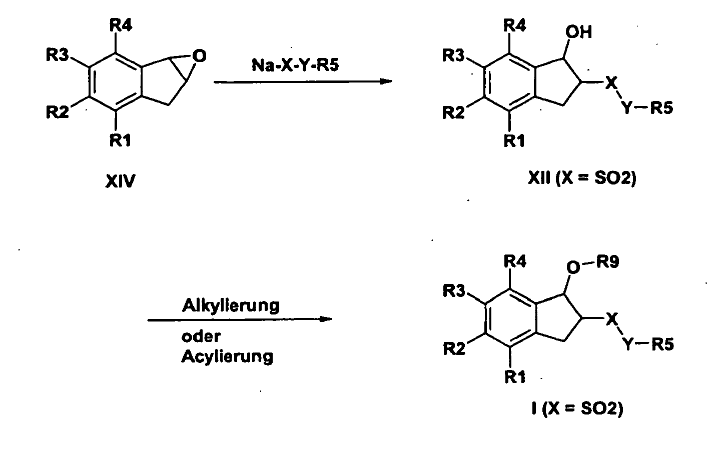

Zur Salzbildung kommen als anorganische Säuren beispielsweise in Betracht: Halogenwasserstoffsäuren wie Chlorwasserstoffsäure und Bromwasserstoffsäure, sowie Schwefelsäure, Phosphorsäure und Amidosulfonsäure.

Als organische Säuren zur Salzbildung seien beispielsweise genannt: Ameisensäure, Essigsäure, Benzoesäure, p-Toluolsulfonsäure, Benzolsulfonsäure, Bernsteinsäure, Fumarsäure, Maleinsäure, Milchsäure, Weinsäure, Zitronensäure, L-Ascorbinsäure, Salizylsäure, Isäthionsäure, Methansulfonsäure, Trifluormethansulfonsäure, 1,2-Benzisothiazol-3(2H)-on, 6-Methyl-1,2,3-oxathiazin-4(3H)-on-2,2-dioxid.

Die nachfolgend aufgeführten Beispiele dienen zur Erläuterung der Erfindung. Die gemessenen Fest-, bzw. Zersetzungspunkte (Fp.) wurden nicht korrigiert und sind generell von der Aufheizgeschwindigkeit abhängig.

**Tabelle 1: Beispiele**

| | | | | | | | | | |
|---|---|---|---|---|---|---|---|---|---|
| 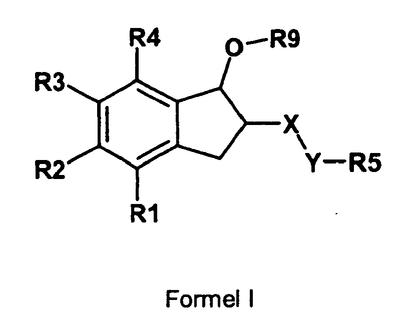 | | | | | | | | | |

| Beispiel | R1 | R2 | R3 | R4 | X | Y | R5 | R9 | Fp. [°C] |
|---|---|---|---|---|---|---|---|---|---|
| 1 | H | Cl | H | H | SO₂ | - | CH₃ | C(O)CH₃ | 136 |
| | | | | | | | | | |
| | | | | | | | | | ¹H nmr (**400 MHz, d₆-dmso, δ _{(CHOCO)} [ppm])** |
| 2 (cis) | H | CI | H | H | SO₂ | - | CH₃ | C(O)-C₆H₄-4-Cl | 6.75 |
| 3 (trans) | H | CI | H | H | SO₂ | - | CH₃ | C(O)-C₆H₄-4-Cl | 6.65 |
| | | | | | | | | | |
| | | | | | | | | | **[MH⁺]** |
| 4 (trans-syn) | H | H | H | H | SO | - | C₆H₅ | CO-CH₃ | 301,12 |
| 5 (trans-anti) | H | H | H | H | SO | - | C₆H₅ | CO-CH₃ | 301,12 |
| 6 (cis-anti) | H | H | H | H | SO | - | C₆H₅ | CO-CH₃ | 301,12 |
| 7 | H | Cl | H | H | S | - | CH₃ | CO-CH₃ | 197,3 (MH⁺-CH₃COO H) |
| 8 | H | N₃ | H | H | SO₂ | - | CH₃ | CO-CH₃ | 236,3 (MH⁺-CH₃COO H) |
| 9 | H | Cl | H | H | SO | - | CH₃ | CO-CH₃ | 213,3 (MH⁺-CH₃COO H) |

Die Verbindungen der Formel I zeichnen sich durch günstige Wirkungen auf den Fettstoffwechsel aus, insbesondere sind sie zur Gewichtreduktion und nach erfolgter Gewichtsreduktion zum Erhalt eines reduzierten Gewichtes bei Säugetieren und als Anorektika geeignet. Die Verbindungen zeichnen sich sich durch ihre geringe Toxizität und ihre geringen Nebenwirkungen aus. Die Verbindungen können allein oder in Kombination mit weiteren gewichtsreduzierenden oder anorektischen Wirkstoffen eingesetzt werden. Solche weiteren anorektischen Wirkstoffe werden z.B. in der Roten Liste, Kapitel 01 unter Abmagerungsmittel/Appetitzügler genannt und können auch solche Wirkstoffe beinhalten, die den Energieumsatz des Organismus erhöhen und damit zu einer Gewichtsreduktion führen oder auch solche, welche den allgemeinen Metabolismus des Organismus so beeinflussen, dass eine erhöhte Kalorienzufuhr nicht zu einer Vergrößerung der Fettdepots und eine normale Kalorienzufuhr zu einer Verringerung der Fettdepots des Organismus führt. Die Verbindungen eignen sich zur Prophylaxe sowie insbesondere zur Behandlung von Übergewicht oder Obesitas

Geeignete Antidiabetika umfassen Insuline, Amylin, GLP-1- und GLP-2-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylfharnstoffe, Biguadine, Meglitinide, Oxadiazolidindione, Thiazolidindione, Glukosidase-Inhibitoren, Glukagon-Rezeptor-Antagonisten, GLP-1-Agonisten, Kaliumkanalöffner wie z.B. diejenigen, die in WO 97/26265 und WO 99/03861 von Novo Nordisk A/S offenbart wurden, Insulin-Sensitizer, Aktivatoren der Insulin Rezeptor Kinase, Inhibitoren von Leberenzymen, die an der Stimulation der Glukoneogenese und/oder Glykogenolyse beteiligt sind, z.B. Inhibitoren der Glycogenphosphorylase, Modulatoren der Glukoseaufnahme und Glukoseausscheidung, den Fettstoffwechsel verändernde Verbindungen wie antihyperlipidämische Wirkstoffe und antilipidämische Wirkstoffe, z.B. HMGCoA-Reduktase-Inhibitoren, Inhibitoren des Cholesteroltransports/der Cholesterolaufnahme, Inhibitoren der Gallensäurerückresorption oder Inhibitoren des mikrosomalen Triglycerid-Transfer Proteins (MTP), Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die vorliegenden Verbindungen in Kombination mit Insulin verabreicht.

Bei einer weiteren Ausführungsform werden die vorliegenden Verbindungen in Kombination mit einem Sulphonylharnstoff wie z.B. Tolbutamid, Glibenclamid, Glimepirid, Glipizid, Gliquidon, Glisoxepid, Glibornurid oder Gliclazid verabreicht.

Bei einer anderen Ausführungsform werden die vorliegenden Verbindungen in Kombination mit einem Biguanid wie z.B. Metformin verabreicht.

Bei wieder einer anderen Ausführungsform werden die vorliegenden Verbindungen in Kombination mit einem Meglitinid wie z.B. Repaglinid verabreicht.

Bei noch einer weiteren Ausführungsform werden die vorliegenden Verbindungen in Kombination mit einem Thiazolidindion wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei noch einer weiteren Ausführungsform werden die vorliegenden Verbindungen in Kombination mit einem Monoamine Oxidase Inhibitor, wie z.B. in WO 01/12176 offenbart, verabreicht. Besonders geeignet sind dabei [3(S),3a(S)]-3-methoxymethyl-7-[4,4,4-trifluorobutoxy]-3,3a,4,5-tetrahydro-1H-oxazolo[3,4-a]quinolin-1-on, (R)-5-(methoxymethyl)-3-[6-(4,4,4-trifluorobutoxy)benzofuran-3-yl]oxazolidin-2-one oder (R)-5-(methoxymethyl)-3-[6-cyclopropylmethoxybenzofuran-3-yl]oxazolidin-2-one.

Bei einer weiteren Ausführungsform werden die vorliegenden Verbindungen in Kombination mit einem α-Glukosidase-Inhibitor wie z.B. Miglitol oder Acarbose verabreicht.

Bei noch einer weiteren Ausführungsform werden die vorliegenden Verbindungen in Kombination mit einem hCNTF (human ciliary neurotrophic factor) oder Derivaten davon wie z.B. CNTF_{AX15} oder modifiziertes CNTF_{AX15}, wie z.B. in Lambert et al., PNAS 98, 4652-4657 offenbart, verabreicht.

Bei einer anderen Ausführungsform werden die vorliegenden Verbindungen in Kombination mit einem Wirkstoff verabreicht, der auf den ATP-abhängigen Kaliumkanal der Betazellen wirkt, wie z.B. Tolbutamid, Glibenclamid, Glimepirid, Glipizid, Gliclazid oder Repaglinid.

Bei noch einer anderen Ausführungsform werden die vorliegenden Verbindungen in Kombination mit einem antihyperlidämischen Wirkstoff oder einem antilipidämischen Wirkstoff wie z.B. Cholestyramin, Colestipol, Clofibrat, Gemfibrozil, Lovastatin, Pravastatin, Simvastatin, Atorvastatin, Cerivastatin, Fluvastatin, Probucol, Ezetimibe oder Dextrothyroxin verabreicht.

Bei einer weiteren Ausführungsform werden die vorliegenden Verbindungen in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Weiterhin können die Verbindungen in Kombination mit einem oder mehreren Antiadiposita oder appetitregulierenden Wirkstoffen verabreicht werden.

Solche Wirkstoffe können ausgewählt werden aus der Gruppe bestehend aus CART-Agonisten, NPY-Antagonisten, Melanocortin 3 oder 4 (MC3 oder MC4) - Agonisten, Melanin-konzentrierendes Hormon (MCH) - Antagonisten, Orexin-Antagonisten, H3-Agonisten, TNF-Agonisten, CRF-Agonisten, CRF BP-Antagonisten, Urocortin-Agonisten, β3-Adrenoceptor Agonisten, CCK-Agonisten, Serotonin-Wiederaufnahme-Inhibitoren, gemischte Sertonin- und Noradrenalin-Wiederaufnahme-Inhibitoren, 5HT-Modulatoren, Bombesin-Agonisten, Galanin-Antagonisten, Glucocorticoid Rezeptor Modulatoren, Wachstumshormon, Wachstumshormon freisetzende Verbindungen, TRH-Agonisten, Modulatoren der Entkopplungsproteine 2 oder 3, Leptin Rezeptor Agonisten, Leptinmimetika, Dopamin-Agonisten (Bromocriptin, Doprexin), Lipase/Amylase-Inhibitoren, Antagonisten des Cannabinoid Rezeptors 1, Modulatoren des die Acylierung stimulierendes Protein (ASP), PPAR-Modulatoren, RXR-Modulatoren oder TR-β-Agonisten.

Bei einer Ausführungsform der Erfindung ist das Antiadipositum Leptin oder modifiziertes Leptin.

Bei einer anderen Ausführungsform ist das Antiadipositum Dexamphetamin oder Amphetamin.

Bei einer anderen Ausführungsform ist das Antiadipositum Fenfluramin oder Dexfenfluramin.

Bei noch einer anderen Ausführungsform ist das Antiadipositum Sibutramin oder die mono- und bisdemethylierten Wirkmetabolite von Sibutramin.

Bei einer weiteren Ausführungsform ist das Antiadipositum Orlistat.

Bei einer anderen Ausführungsform ist das Antiadipositum Mazindol, Diethylpropion oder Phentermin.

Weiterhin können die vorliegenden Verbindungen in Kombination mit einem oder mehreren antihypertensiven Wirkstoffen verabreicht werden. Beispiele für antihypertensive Wirkstoffe sind Betablocker wie Alprenolol, Atenol, Timolol, Pindolol, Propanolol und Metoprolol, ACE (Angiotensin Converting Enzym)-Hemmer wie z.B. Benazepril, Captopril, Enalapril, Fosinopril, Lisinopril, Quinapril und Rampril, Calciumkanal-Blocker wie Nifedipin, Felodipin, Nicardipin, Isradipin, Nimodipin, Diltiazem und Verapamil, sowie Alphablocker wie Doxazosin, Urapidil, Prazosin und Terazosin. Weiterhin kann verwiesen werden auf Remington: The Science and Practice of Pharmacy, 19. Auflage, Gennaro, Hrsg., Mack Publishing Co., Easton, PA, 1995.

Die folgenden Zubereitungen dienen zur Erläuterung der Erfindung.

### Beispiel A

| Gelatineweichkapseln, enthaltend 100 mg Wirkstoff pro Kapsel: | |
|---|---|
| | pro Kapsel |
| Wirkstoff | 100 mg |
| aus Kokosfett fraktioniertes | |
| Triclycerid-Gemisch | 400 mg |
| Kapselinhalt | 500 mg |

### Beispiel B

| Emulsion, enthaltend 60 mg Wirkstoff pro 5 ml: | |
|---|---|
| | pro 100 ml Emulsion |
| Wirkstoff | 1,2 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

### Beispiel C

| Rektale Arzneiform, enthaltend 40 mg Wirkstoff pro Suppositorium: | |
|---|---|
| | pro Suppositorium |
| Wirkstoff | 40 mg |
| Suppositoriengrundmasse | ad 2 g |

### Beispiel D

| Tabletten, enthaltend 40 mg Wirkstoff pro Tablette: | |
|---|---|
| | pro Tablette |
| Lactose | 600 mg |
| Maisstärke | 300 mg |
| lösliche Stärke | 20 mg |
| Magnesiumstearat | 40 mg |
| | 1000 mg |

### Beispiel E

| Dragees, enthaltend 50 mg Wirkstoff pro Dragees: | |
|---|---|
| | pro Dragee |
| Wirkstoff | 50 mg |
| Maisstärke | 100 mg |
| Lactose | 60 mg |
| sec. Calciumphosphat | 30 mg |
| lösliche Stärke | 5 mg |
| Magnesiumstearat | 10 mg |
| kolloidale Kieselsäure | 5 mg |
| | 260 mg |

### Beispiel F

Für die Herstellung des Inhalts von Hartgelatinekapseln eignen sich die folgenden Rezepturen:

| | | |
|---|---|---|
| a) | Wirkstoff | 100 mg |
| | Maisstärke | 300 mg |
| | | 400 mg |
| b) | Wirkstoff | 140 mg |
| | Milchzucker | 180 mg |
| | Maisstärke | 180 mg |
| | | 500 mg |

### Beispiel G

| Tropfen können nach folgender Rezeptur hergestellt werden (100 mg Wirkstoff in 1 ml = 20 Tropfen): | |
|---|---|
| Wirkstoff | 10 g |
| Benzoesäuremethylester | 0,07 g |
| Benzoesäureethylester | 0,03 g |
| Ethanol 96 %ig | 5 ml |
| entmineralisiertes Wasser | ad 100 ml |

Die Wirksamkeit der Verbindungen der Formel I wurde wie folgt getestet:

### Biologisches Prüfmodell:

Die Prüfung der anorektischen Wirkung erfolgte an weiblichen NMRI Mäusen. Nach 24stündigem Futterentzug wurde das Testpräparat intraperitoneal (ip) oder über eine Schlundsonde (po) verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 7 Stunden lang bestimmt und das Allgemeinbefinden der Tiere beobachtet. Der gemessene Milchverbrauch wurde mit dem unbehandelter Kontrolltieren verglichen.

**Tabelle 2: Anorektische Wirkung, gemessen als Reduktion des kumulierten Milchkonsums behandelter im Vergleich zu dem unbehandelter Tiere.**

| Verbindung/Beispiel 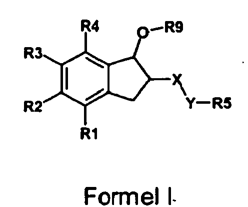 | Dosis [mg/kg] | Anzahl der Tiere / Kumulierter Milchkonsum der behandelten Tiere N / [ml] | Anzahl der Tiere / Kumulierter Milchkonsum der unbehandelten Kontrolltiere N / [ml] | Reduktion des kumulierten Milchkonsums in % der Kontrolle |
|---|---|---|---|---|
| | | | | |
| Beispiel 1 | 10 | 5/0,28 | 5/1,50 | 81 |

Aus der Tabelle ist abzulesen, daß die Verbindungen der Formel I eine sehr gute anorektische Wirkung zeigen.

Nachfolgend wird die Herstellung einiger Beispiele detailliert beschrieben, die übrigen Verbindungen der Formel I wurden analog erhalten:

### Beispiel 1:

### 1-Acetoxy-5-Chlor-2-methylsulfonyl-indan:

### 1. 5-Chlor-2-methylsulfanyl-indan-1-on:

0.98 g (4-mmol) 2-Brom-5-chlor-indan-1-on und 0.42 g (6 mmol) Natriumthiomethylat werden in 5 ml Ethanol suspendiert, 30 Minuten im Ultraschallbad behandelt und anschließend 90 Minuten bei Raumtemperatur gerührt. Die Reaktionsmischung wird im Vakuum eingeengt und über Kieselgel mit Toluol/Essigsäureethylester 10/1 chromatographiert. Die Eluate werden im Vakuum eingeengt und liefern 0.63 g 5-Chlor-2-methylsulfanyl-indan-1-on mit dem Schmelzpunkt 90°C.

### 2. 5-Chlor-2-methylsulfonyl-indan-1-on:

0.5 g (2.35 mmol) 5-Chlor-2-methylsulfanyl-indan-1-on werden in 10 ml Methanol gelöst; bei 0°C wird eine Lösung aus 4.33 g (7.05 mmol) 2KHSO₅ x KHSO₄ x K₂SO₄ in 10 ml Wasser zugetropft. Die Mischung wird 5 h bei Raumtemperatur gerührt; das Methanol wird abdestilliert und der wässrige Rückstand wird mit Dichlormethan extrahiert. Die organische Phase wird abgetrennt, über MgSO₄ getrocknet, filtriert und im Vakuum eingeengt. Man erhält 0.5 g 5-Chlor-2-methansulfonyl-indan-1-on mit dem Schmelzpunkt 197°C.

### 3. 5-Chlor-2-methylsulfonyl-indan-1-ol:

0.489 g (2 mmol) 5-Chlor-2-methylsulfonyl-indan-1-on und 0.095 g (2.5 mmol) Natriumborhydrid werden in 10 ml Ethanol supendiert und 4 h ins Ultraschallbad gestellt. Danach wird die Reaktionsmischung mit 2N HCl angesäuert und gerührt. Das Ethanol wird abdestilliert, der Rückstand mit Wasser und Dichlormethan versetzt, und die Mischung mit Dichlormethan extrahiert. Die organische Phase wird abgetrennt, über MgSO₄ getrocknet, filtriert, eingeengt und der Rückstand über Kieselgel mit Dichlormethan/Methanol 20/1 chromatographisch gereinigt. Man erhält 5-Chlor-2-methylsulfonyl-indan-1-ol mit dem Schmelzpunkt 163°C.

### 4. 1-Acetoxy-5-Chlor-2-methytsulfonyl-indan:

0.247 g (1 mmol) 5-Chlor-2-methylsulfonyl-indan-1-ol werden in 5 ml Dichlormethan gelöst. Die Lösung wird mit 1 ml Pyridin und 1 ml Essigsäureanhydrid versetzt und die Reaktionsmischung wird anschließend 2 Tage bei Raumtemperatur gerührt. Die Reaktionslösung wird vollständig eingeengt, der Rückstand mit Dichlormethan aufgenommen und mit verdünnter Zitronensäure ausgeschüttelt. Die organische Phase wird abgetrennt, eingeengt und chroamtographisch (Kieselgel; Dichlormethan/Methanol 20/1) gereinigt. Man erhält 1-Acetoxy-5-Chlor-2-methylsulfonyl-indan mit dem Schmelzpunkt 136°C.

### Beispiel 2:

Der Umsatz von 5-Chlor-2-methylsulfonyl-indan-1-ol mit 4-Chlorbenzoylchlorid liefert cis- und trans-4-Chlor-benzoesäure-5-chlor-2-methansulfonyl-indan-1-yl ester.

### Beispiel 4 - 9:

Der Umsatz der entsprechenden Alkohole mit Essigsäurechlorid liefert die beschriebenen O-Acetylderivate.

## Patentansprüche

1. Verwendung der Verbindungen der Formel I, 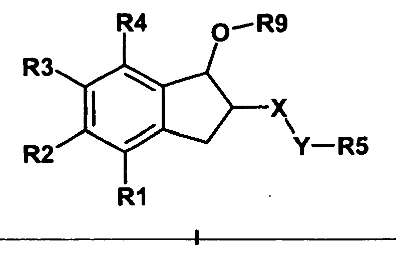 worin bedeuten
R1, R2, R3, R4 unabhängig voneinander H, F, CI, Br, J, CN; N₃, NO₂, OH, O(C₁-C₈)-Alkyl, O(C₃-C₈)-Cycloalkyl, O-CH₂-phenyl, O-phenyl, O-CO-(C₁-C₈)-Alkyl, O-CO-(C₃-C₈)-Cycloalkyl, S(O)₀₋₂(C₁-C₈)-Alkyl, S(O)₀₋₂(C₃-C₈)-Cycloalkyl, NH₂, NH-(C₁-C₈)-Alkyl, NH-(C₃-C₈)-Cycloalkyl, N[(C₁-C₈)-Alkyl]₂, N[(C₃-C₈)-Cycloalkyl]₂, NH-CO-(C₁-C₈)-Alkyl, NH-CO-(C₃-C₈)-Cycloalkyl; SO₃H, SO₂-NH₂, SO₂-NH-(C₁-C₈)-Alkyl, SO₂-NH-(C₃-C₈)-Cycloalkyl, NH-SO₂-NH₂, NH-SO₂-(C₁-C₈)-Alkyl, NH-SO₂-(C₃-C₈)-Cycloalkyl, O-CH₂-COOH, O-CH₂-CO-O(C₁-C₈)-Alkyl, O-CH₂-COOH, O-CH₂-CO-O(C₁-C₈)-Alkyl, COOH, CO-O(C₁-C₈)-Alkyl, CO-O-(C₃-C₈)-Cycloalkyl, CO-NH₂, CO-NH(C₁-C₈)-Alkyl, CO-N[(C₁-C₈)-Alkyl]₂, (C₁-C₈)-Alkyl, (C₃-C₈)-Cycloalkyl, (C₂-C₈)-Alkenyl, (C₂-C₈)-Alkinyl, wobei in den Alkyl-, Alkenyl- und Alkinylgruppen jeweils ein bis zu sieben Wasserstoffatome durch Fluor ersetzt sein können, oder ein Wasserstoff durch OH, OC(O)CH₃, O-CH₂-Ph, NH₂, NH-CO-CH₃ oder N(COOCH₂Ph)₂ ersetzt sein kann;
Phenyl, 1- oder 2-Naphthyl,
5-Tetrazolyl, 1-[(C₁-C₆)-Alkyl]-5-Tetrazolyl, 2-[(C₁-C₆)-Alkyl]-5-Tetrazolyl,
1-Imidazolyl,
1-oder 4-[1,2,4]Triazolyl,
2- oder 3-Thienyl,
2- oder 3-Furyl,
2-, 3- oder 4-Pyridyl,
2-, 4- oder 5-Oxazolyl,
3-, 4- oder 5-Isoxazolyl,
2-, 4- oder 5-Thiazolyl,
3-, 4- oder 5-Isothiazolyl
wobei der Arylrest oder Heterocyclus bis zu zweifach mit F, Cl, Br, CN, OH, (C₁-C₄)-Alkyl, CF₃, O-(C₁-C₄)-Alkyl, S(O)₀₋₂(C₁-C₆)-Alkyl, NH₂, NH-SO₂-(C₁-C₄)-Alkyl, COOH, CO-O-(C₁-C₄)-Alkyl, CO-NH₂ substituiert sein kann und in den Alkylgruppen ein bis sieben Wasserstoffatome durch Fluor ersetzt sein können;
oder R2 und R3 bilden gemeinsam die Gruppe -O-CH₂-O-;
X S, SO, SO₂;
Y (CH₂)ₚ, wobei p gleich 0,1, 2 oder 3 sein kann;
R5 CF3, (C₁-C₁₈)-Alkyl, (C₃-C₈)-Cycloalkyl, wobei in den Alkylgruppen ein bis sieben Wasserstoffatome durch Fluor ersetzt sein können;
(CH₂)ᵣ-COR6, wobei r = 1-6 sein kann und R6 gleich OH, O-(C₁-C₆)-Alkyl oder NH₂ sein kann;
CH₂-CH(NHR7)-COR8, wobei R7 gleich H oder C(O)-(C₁-C₄)-Alkyl und R8 gleich OH, O-(C₁-C₆)-Alkyl oder NH₂ sein kann;
Phenyl, 1- oder 2-Naphthyl, Biphenyl oder ein Heterocyclischer Rest, wobei die Ringe oder Ringsysteme bis zu zweifach substituiert sein können mit F, Cl, Br, J, CN, OH, O(C₁-C₈)-Alkyl, O(C₃-C₈)-Cycloalkyl, O-CO-(C₁-C₈)-Alkyl, O-CO-(C₃-C₈)-Cycloalkyl, S(O)₀₋₂(C₁-C₈)-Alkyl, S(O)₀₋₂(C₃-C₈)-Cycloalkyl, NH₂, NH-(C₁-C₈)-Alkyl, NH-(C₃-C₈)-Cycloalkyl, N[(C₁-C₈)-Alkyl]₂, N[(C₃-C₈)-Cycloalkyl]₂, NH-CO-(C₂-C₈)-Alkyl, NH-CO-(C₃-C₈)-Cycloalkyl; SO₃H, SO₂-NH₂, SO₂-NH-(C₁-C₈)-Alkyl, SO₂-NH-(C₃-C₈)-Cycloalkyl; NH-SO₂-NH₂, NH-SO₂-(C₁-C₈)-Alkyl, NH-SO₂-(C₃-C₈)-Cycloalkyl, O-CH₂-COOH, O-CH₂-CO-O(C₁-C₈)-Alkyl, COOH, CO-O(C₁-C₈)-Alkyl, CO-O-(C₃-C₈)-Cycloalkyl, CO-NH₂, CO-NH(C₁-C₈)-Alkyl, CO-N[(C₁-C₈)-Alkyl]₂, (C₁-C₈)-Alkyl, (C₃-C₈)-Cycloalkyl, wobei in den Alkylgruppen jeweils ein bis zu sieben Wasserstoffatome durch Fluor ersetzt sein können;
R9 (C₁-C₁₂)-Alkyl, (C₃-C₈)-Cycloalkyl, wobei in den Alkylresten ein bis sieben Wasserstoffatome durch Fluor ersetzt sein können; CO-O(C₁-C₆)-Alkyl, CO-O(C₃-C₈)-Cycloalkyl, C(O)-(C₁-C₈)-Alkyl, C(O)-(C₃-C₈)-Cycloalkyl, C(O)-Phenyl, C(O)-CH(NHR12)-(C₁-C₈)-Alkyl, Phenyl, 1-oder 2-Naphthyl, Biphenyl, 2-, 3- oder 4-Pyridyl, wobei die Aryl- oder Heteroarylreste bis zu zweifach mit F, Cl, Br, CN, OH, (C₁-C₄)-Alkyl, CF₃, O-(C₁-C₄)-Alkyl, S(O)₀₋₂(C₁-C₆)-Alkyl, NH₂, NH-SO₂-(C₁-C₄)-Alkyl, -CH₂-COOH, O-CH₂-CO-O(C₁-C₈)-Alkyl, COOH, CO-O-(C₁-C₄)-Alkyl, CO-NH₂ substituiert sein können; (CH₂)-R10;
(CH₂)ₛ-R11, wobei s = 2 oder 3 sein kann;
R10 (C₁-C₁₂)-Alkyl, (C₃-C₈)-Cycloalkyl, wobei in den Alkylresten ein bis sieben Wasserstoffatome durch Fluor ersetzt sein können; COOH, CONH₂, CO-O(C₁-C₆)-Alkyl, CO-O(C₃-C₈)-Cycloalkyl; Phenyl, 1-oder 2-Naphthyl, Biphenyl, 2-, 3- oder 4-Pyridyl, 2- oder 3-Furyl oder 2- oder3-Thienyl sein kann, wobei die Aryl- oder Heteroarylreste bis zu zweifach mit F, Cl, Br, CN, OH, (C₁-C₄)-Alkyl, CF₃, O-(C₁-C₄)-Alkyl, S(O)₀₋₂(C₁-C₆)-Alkyl, NH₂, NH-SO₂-(C₁-C₄)-Alkyl, O-CH₂-COOH, O-CH₂-CO-O(C₁-C₈)-Alkyl, COOH, CO-O-(C₁-C₄)-Alkyl, CO-NH₂ substituiert sein können;
R11 (C₁-C₁₂)-Alkyl, (C₃-C₈)-Cycloalkyl, wobei in den Alkylresten ein bis sieben Wasserstoffatome durch Fluor ersetzt sein können; COOH, CONH₂, CO-O(C₁-C₆)-Alkyl, CO-O(C₃-C₈)-Cycloalkyl; Phenyl, 1-oder 2-Naphthyl, Biphenyl, 2-, 3-oder 4-Pyridyl, 2- oder 3-Furyl, 2- oder 3-Thienyl oder 1-Imidazolyl,
wobei die Aryl- oder Heteroarylreste bis zu zweifach mit F, Cl, Br, CN, OH, (C₁-C₄)-Alkyl, CF₃, O-(C₁-C₄)-Alkyl, S(O)₀₋₂(C₁-C₆)-Alkyl, NH₂, NH-SO₂-(C₁-C₄)-Alkyl, O-CH₂-COOH, O-CH₂-CO-O(C₁-C₈)-Alkyl, COOH, CO-O-(C₁-C₄)-Alkyl, CO-NH₂ substituiert sein können;
R12 H, C(O)-(C₁-C₆)-Alkyl;
sowie derer physiologisch verträgliche Salze zur Herstellung eines Medikaments zur Gewichtsreduktion von Säugetieren.

2. Verwendung gemäß Anspruch 1 zur Herstellung eines Medikaments zur Prophylaxe oder Behandlung von Obesitas.

3. Verwendung gemäß Anspruch 1 in Kombination mit einem oder mehren gewichtsreduzierenden Wirkstoffen zur Herstellung eines Medikaments zur Gewichtsreduktion von Säugetieren.

4. Verwendung gemäß Anspruch 1 in Kombination mit einem oder mehren gewichtsreduzierenden Wirkstoffen zur Herstellung eines Medikaments zur Prophylaxe oder Behandlung von Obesitas.

5. Verwendung gemäß Anspruch 1 in Kombination mit Cathine, Phenylpropanolamine, Amfepramone, Mefenorex, Ephedrine, Leptin, Dexamphetamine, Amphetamine, Fenfluramine, Dexfenfluramine, Sibutramine, Orlistat, Mazindol oder Phentermine oder deren Salze zur Herstellung eines Medikaments zur Gewichtsreduktion von Säugetieren.

6. Verwendung gemäß Anspruch 1 in Kombination mit Cathine, Phenylpropanolamine, Amfepramon, Mefenorex, Ephedrine, Leptin, Dexamphetamine, Amphetamine, Fenfluramine, Dexfenfluramine, Sibutramine, Orlistat, Mazindol oder Phentermine oder deren Salze zur Herstellung eines Medikaments zur Prophylaxe oder Behandlung von Obesitas.

## Claims

1. The use of the compounds of the formula I, 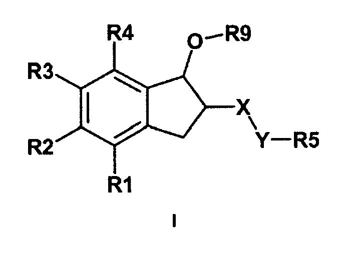 in which
R1, R2, R3, R4 independently of one another are H, F, Cl, Br, I, CN; N₃, NO₂, OH, O(C₁-C₈)-alkyl, O(C₃-C₈)-cycloalkyl, O-CH₂-phenyl, O-phenyl, O-CO-(C₁-C₈)-alkyl, O-CO-(C₃-C₈)-cycloalkyl, S(O)₀₋₂(C₁-C₈)-alkyl, S(O)₀₋₂(C₃-C₈)-cycloalkyl, NH₂, NH-(C₁-C₈)-alkyl, NH-(C₃-C₈)-cycloalkyl, N[(C₁-C₈)-alkyl]₂, N[(C₃-C₈)-cycloalkyl]₂, NH-CO-(C₁-C₈)-alkyl, NH-CO-(C₃-C₈)-cycloalkyl; SO₃H, SO₂-NH₂, SO₂-NH-(C₁-C₈)-alkyl, SO₂-NH-(C₃-C₈)-cycloalkyl, NH-SO₂-NH₂, NH-SO₂-(C₁-C₈)-alkyl, NH-SO₂-(C₃-C₈)-cycloalkyl, O-CH₂-COOH, O-CH₂-CO-O(C₁-C₈)-alkyl, O-CH₂-COOH, O-CH₂-CO-O(C₁-C₈)-alkyl, COOH, CO-O(C₁-C₈)-alkyl, CO-O-(C₃-C₈)-cycloalkyl, CO-NH₂, CO-NH(C₁-C₈)-alkyl, CO-N[(C₁-C₈)-alkyl]₂, (C₁-C₈)-alkyl, (C₃-C₈)cycloalkyl, (C₂-C₈)-alkenyl, (C₂-C₈)-alkynyl, where in the alkyl, alkenyl and alkynyl groups in each case one to seven hydrogen atoms may be replaced by fluorine, or one hydrogen may be replaced by OH, OC(O)CH₃, O-CH₂-Ph, NH₂, NH-CO-CH₃ or N(COOCH₂Ph)₂; phenyl, 1- or 2-naphthyl,
5-tetrazolyl, 1-[(C₁-C₆)-alkyl]-5-tetrazolyl, 2-[(C₁-C₆)-alkyl]-5-tetrazolyl,
1-imidazolyl,
1- or 4-[1,2,4]-triazolyl,
2- or 3-thienyl,
2- or 3-furyl,
2-, 3- or 4-pyridyl,
2-, 4- or 5-oxazolyl,
3-, 4- or 5-isoxazolyl,
2-, 4- or 5-thiazolyl,
3-, 4- or 5-isothiazolyl,
where the aryl radical or heterocycle may be substituted up to two times by F, Cl, Br, CN, OH, (C₁-C₄)-alkyl, CF₃, O-(C₁-C₄)-alkyl, S(O)₀₋₂(C₁-C₆)-alkyl, NH₂, NH-SO₂-(C₁-C₄)-alkyl, COOH, CO-O-(C₁-C₄)-alkyl, CO-NH₂ and where in the alkyl groups one to seven hydrogen atoms may be replaced by fluorine;
or R2 and R3 together form the group -O-CH₂-O-;
X is S, SO, SO₂;
Y is (CH₂)ₚ, where p may be 0, 1, 2 or 3;
R5 is CF₃, (C₁-C₁₈)-alkyl, (C₃- C₈)-cycloalkyl, where in the alkyl groups one to seven hydrogen atoms may be replaced by fluorine;
(CH₂)ᵣ-COR6, where r may be 1-6 and R6 may be OH, O-(C₁-C₆)-alkyl or NH₂;
CH₂-CH(NHR7)-COR8, where R7 may be H or C(O)-(C₁-C₄)-alkyl and R8 may be OH, O-(C₁-C₆)-alkyl or NH₂;
phenyl, 1- or 2-naphthyl, biphenyl or a heterocyclic radical, where the rings or ring systems may be substituted up to two times by
F, Cl, Br, I, CN, OH, O(C₁-C₈)-alkyl, O(C₃-C₈)-cycloalkyl, O-CO-(C₁-C₈)-alkyl, O-CO-(C₃-C₈)-cycloalkyl, S(O)₀₋₂(C₁-C₈)-alkyl, S(O)₀₋₂(C₃-C₈)-cycloalkyl, NH₂, NH-(C₁-C₈)-alkyl, NH-(C₃-C₈)-cycloalkyl, N[(C₁-C₈)-alkyl]₂, N[(C₃-C₈)-cycloalkyl]₂, NH-CO-(C₂-C₈)-alkyl, NH-CO-(C₃-C₈)-cycloalkyl; SO₃H, SO₂-NH₂, SO₂-NH-(C₁-C₈)-alkyl, SO₂-NH-(C₃-C₈)-cycloalkyl; NH-SO₂-NH₂, NH-SO₂-(C₁-C₈)-alkyl, NH-SO₂-(C₃-C₈)-cycloalkyl, O-CH₂-COOH, O-CH₂-CO-O(C₁-C₈)-alkyl, COOH, CO-O(C₁-C₈)-alkyl, CO-O-(C₃-C₈)-cycloalkyl, CO-NH₂, CO-NH(C₁-C₈)-alkyl, CO-N[(C₁-C₈)-alkyl]₂, (C₁-C₈)-alkyl, (C₃-C₈)-cycloalkyl, where in the alkyl groups in each case one to seven hydrogen atoms may be replaced by fluorine;
R9 is (C₁-C₁₂)-alkyl, (C₃-C₈)-cycloalkyl, where in the alkyl radicals one to seven hydrogen atoms may be replaced by fluorine; CO-O(C₁-C₆)-alkyl, CO-O(C₃-C₈)-cycloalkyl, C(O)-(C₁-C₈)-alkyl, C(O)-(C₃-C₈)-cycloalkyl, C(O)-phenyl, C(O)-CH(NHR12)-(C₁-C₈)-alkyl, phenyl, 1- or 2-naphthyl, biphenyl, 2-, 3- or 4-pyridyl, where the aryl or heteroaryl radicals may be substituted up to two times by F, Cl, Br, CN, OH, (C₁-C₄)-alkyl, CF₃, O-(C₁-C₄)-alkyl, S(O)₀₋₂(C₁-C₆)-alkyl, NH₂, NH-SO₂-(C₁-C₄)-alkyl, -CH₂-COOH, O-CH₂-CO-O(C₁-C₈)-alkyl, COOH, CO-O-(C₁-C₄)-alkyl, CO-NH₂; (CH₂)-R10;
(CH₂)ₛ-R11, where s may be 2 or 3;
R10 e (C₁-C₁₂)-alkyl, (C₃-C₈)-cycloalkyl, where in the alkyl radicals one to seven hydrogen atoms may be replaced by fluorine; is COOH, CONH₂, CO-O(C₁-C₆)-alkyl, CO-O(C₃-C₈)-cycloalkyl;
phenyl, 1- or 2-naphthyl, biphenyl, 2-, 3- or 4-pyridyl, 2- or 3-furyl or 2- or 3-thienyl, where the aryl or heteroaryl radicals may be substituted up to two times by
F, Cl, Br, CN, OH, (C₁-C₄)-alkyl, CF₃, O-(C₁-C₄)-alkyl, S(O)₀₋₂(C₁-C₆)-alkyl, NH₂, NH-SO₂-(C₁-C₄)-alkyl, O-CH₂-COOH, O-CH₂-CO-O(C₁-C₈)-alkyl, COOH, CO-O-(C₁-C₄)-alkyl, CO-NH₂;
R11 is (C₁-C₁₂)-alkyl, (C₃-C₈)-cycloalkyl, where in the alkyl radicals one to seven hydrogen atoms may be replaced by fluorine; COOH, CONH₂, CO-O(C₁-C₆)-alkyl, CO-O(C₃-C₈)-cycloalkyl; phenyl, 1- or 2-naphthyl, biphenyl, 2-, 3- or 4-pyridyl, 2- or 3-furyl, 2- or 3-thienyl or 1-imidazolyl,
where the aryl or heteroaryl radicals may be substituted up to two times by
F, Cl, Br, CN, OH, (C₁-C₄)-alkyl, CF₃, O-(C₁-C₄)-alkyl, S(O)₀₋₂(C₁-C₆)-alkyl, NH₂, NH-SO₂-(C₁-C₄)-alkyl, O-CH₂-COOH, O-CH₂- CO-O(C₁-C₈)-alkyl, COOH, CO-O-(C₁-C₄)-alkyl, CO-NH₂;
R12 is H, C(O)-(C₁-C₆)-alkyl;
and their physiologically acceptable salts for preparing a medicament for reducing weight in mammals.

2. The use as claimed in claim 1 for the preparation of a medicament for the prophylaxis or treatment of obesity.

3. The use as claimed in claim 1 in combination with one or more active compounds suitable for reducing weight, for the preparation of a medicament for reducing weight in mammals.

4. The use as claimed in claim 1 in combination with one or more active compounds suitable for reducing weight, for the preparation of a medicament for the prophylaxis or treatment of obesity.

5. The use as claimed in claim 1 in combination with cathine, phenylpropanolamine, amfepramone, mefenorex, ephedrine, leptin, dexamphetamine, amphetamine, fenfluramine, dexfenfluramine, sibutramine, orlistat, mazindol or phentermine or their salts for preparing a medicament for reducing weight in mammals.

6. The use as claimed in claim 1 in combination with cathine, phenylpropanolamine, amfepramone, mefenorex, ephedrine, leptin, dexamphetamine, amphetamine, fenfluramine, dexfenfluramine, sibutramine, orlistat, mazindol or phentermine or their salts for preparing a medicament for the prophylaxis or treatment of obesity.

## Revendications

1. Utilisation des composés de formule I, 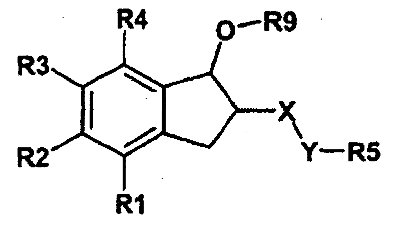 dans laquelle
R1, R2, R3, R4 signifient, indépendamment l'un de l'autre H, F, Cl, Br, I, CN ; N₃, NO₂, OH, O(C₁-C₈)-alkyle, O(C₃-C₈)-cycloalkyle, O-CH₂-phényle, O-phényle, O-CO-(C₁-C₈)-alkyle, O-CO-(C₃-C₈)-cycloalkyle, S (O)₀₋₂(C₁-C₈) -alkyle, S (O)₀₋₂(C₃-C₈)-cycloalkyle, NH₂, NH- (C₁-C₈)-alkyle, NH-(C₃-C₈)-cycloalkyle, N[(C₁-C₈)-alkyle]₂, N[(C₃-C₈)-cycloalkyle]₂, NH-CO-(C₁-C₈) -alkyle, NH-CO- (C₃-C₈)-cycloalkyle ; SO₃H, SO₂-NH₂, SO₂-NH- (C₁-C₈)-alkyle, SO₂-NH-(C₃-C₈)-cycloalkyle, NH-SO₂-NH₂, NH-SO₂-(C₁-C₈)-alkyle, NH-SO₂- (C₃-C₈) -cycloalkyle, O-CH₂-COOH, O-CH₂-CO-O(C₁-C₈)-alkyle, O-CH₂-COOH, O-CH₂-CO-O(C₁-C₈)-alkyle, COOH, CO-O(C₁-C₈)-alkyle, CO-O-(C₃-C₈)-cycloalkyle, CO-NH₂, CO-NH(C₁-C₈)-alkyle, CO-N[(C₁-C₈)-alkyle]₂, (C₁-C₈)-alkyle, (C₃-C₈) -cycloalkyle, (C₂-C₈)-alcényle, (C₂-C₈)-alcynyle, où, dans les groupes alkyle, alcényle et alcynyle, à chaque fois un à sept atomes d'hydrogène peuvent être remplacés par fluor, ou un hydrogène peut être remplacé par OH, OC(O)CH₃, O-CH₂-Ph, NH₂, NH-CO-CH₃
ou N(COOCH₂Ph)₂;
phényle, 1-naphtyle ou 2-naphtyle,
5-tétrazolyle, 1-[(C₁-C₆)-alkyl]-5-tétrazolyle, 2-[(C₁-C₆)-alkyl]-5-tétrazolyle,
1-imidazolyle,
1-[1,2,4]triazolyle ou 4-[1,2,4]triazolyle,
2-thiényle ou 3-thiényle,
2-furyle ou 3-furyle,
2-pyridyle, 3-pyridyle ou 4-pyridyle,
2-oxazolyle, 4-oxazolyle ou 5-oxazolyle,
3-isoxazolyle, 4-isoxazolyle ou 5-isoxazolyle,
2-thiazolyle, 4-thiazolyle ou 5-thiazolyle,
3-isothiazolyle, 4-isothiazolyle ou 5-isothiazolyle
le radical aryle ou l'hétérocycle pouvant être jusqu'à disubstitué par
F, Cl, Br, CN, OH, (C₁-C₄)-alkyle, CF₃, O-(C₁-C₄)-alkyle, S (O)₀₋₂(C₁-C₆)-alkyle, NH₂, NH-SO₂-(C₁-C₄)-alkyle, COOH, CO-O-(C₁-C₄)-alkyle, CO-NH₂ et, dans les groupes alkyle, un à sept atomes d'hydrogène pouvant être remplacés par fluor ;
ou R2 et R3 forment ensemble le groupe -O-CH₂-O- ;
X signifie S, SO, SO₂ ;
Y signifie (CH₂)ₚ, où p peut être égal à 0, 1, 2 ou 3 ;
R5 signifie CF₃, (C₁-C₁₈)-alkyle, (C₃-C₈)-cycloalkyle, où, dans les groupes alkyle, un à sept atomes d'hydrogène peuvent être remplacés par fluor ;
(CH₂)ᵣ-COR6, où r peut être = 1-6 et R6 peut représenter OH, O-(C₁-C₆)-alkyle ou NH₂ ;
CH₂-CH(NHR7)-COR8, où R7 peut représenter H ou C(O)-(C₁-C₄)-alkyle et R8 peut représenter OH, O-(C₁-C₆)-alkyle ou NH₂ ;
phényle, 1-naphtyle ou 2-naphtyle, biphényle ou un radical hétérocyclique, où les cycles ou les systèmes cycliques peuvent être jusqu'à disubstitués par F, Cl, Br, I, CN, OH, O(C₁-C₈)-alkyle, O (C₃-C₈) -cycloalkyle, O-CO-(C₁-C₈) -alkyle, O-CO-(C₃-C₈) -cycloalkyle, S(O)₀₋₂(C₁-C₈)-alkyle, S (O)₀₋₂(C₃-C₈) -cycloalkyle, NH₂, NH-(C₁-C₈)-alkyle, NH- (C₃-C₈)-cycloalkyle, N [(C₁-C₈) -alkyle]₂, N [(C₃-C₈)-cycloalkyle]₂, NH-CO-(C₂-C₈)-alkyle, NH-CO-(C₃-C₈)-cycloalkyle ; SO₃H, SO₂-NH₂, SO₂-NH-(C₁-C₈)-alkyle, SO₂-NH-(C₃-C₈)-cycloalkyle ; NH-SO₂-NH₂, NH-SO₂- (C₁-C₈) -alkyle, NH-SO₂- (C₃-C₈) -cycloalkyle, O-CH₂-COOH, O-CH₂-CO-O(C₁-C₈)-alkyle, COOH, CO-O(C₁-C₈)-alkyle, CO-O-(C₃-C₈)-cycloalkyle, CO-NH₂, CO-NH (C₁-C₈) -alkyle, CO-N[(C₁-C₈)-alkyle]₂, (C₁-C₈)-alkyle, (C₃-C₈)-cycloalkyle, où, dans les groupes alkyle, à chaque fois un à sept atomes d'hydrogène peuvent être remplacés par fluor ;
R9 signifie (C₁-C₁₂)-alkyle, (C₃-C₈)-cycloalkyle, où, dans les radicaux alkyle, un à sept atomes d'hydrogène peuvent être remplacés par fluor ; CO-O(C₁-C₆)-alkyle, CO-O(C₃-C₈)-cycloalkyle, C(O)-(C₁-C₈)-alkyle, C(O)-(C₃-C₈)-cycloalkyle, C(O)-phényle, C(O)-CH(NHR12)-(C₁-C₈)-alkyle, phényle, 1-naphtyle ou 2-naphtyle, biphényle, 2-pyridyle, 3-pyridyle ou 4-pyridyle, où les radicaux aryle ou hétéroaryle peuvent être jusqu'à disubstitués par F, Cl, Br, CN, OH, (C₁-C₄)-alkyle, CF₃, O-(C₁-C₄)-alkyle, S (O) ₀₋₂ (C₁-C₆)-alkyle, NH₂, NH-SO₂-(C₁-C₄)-alkyle, -CH₂-COOH, O-CH₂-CO-O(C₁-C₈) -alkyle, COOH, CO-O-(C₁-C₄)-alkyle, CO-NH₂ ;
(CH₂)-R10 ;
(CH₂)ₛ-R11, où s peut être = 2 ou 3 ;
R10 signifie (C₁-C₁₂)-alkyle, (C₃-C₈) -cycloalkyle, où, dans les radicaux alkyle, un à sept atomes d'hydrogène peuvent être remplacés par fluor ; COOH, CONH₂, CO-O (C₁-C₆)-alkyle, CO-O (C₃-C₈)-cycloalkyle ; phényle, 1-naphtyle ou 2-naphtyle, biphényle, 2-pyridyle, 3-pyridyle ou 4-pyridyle, 2-furyle ou 3-furyle ou 2-thiényle ou 3-thiényle, les radicaux aryle ou hétéroaryle pouvant être jusqu'à disubstitués par F, C1, Br, CN, OH, (C₁-C₄)-alkyle, CF₃, O- (C₁-C₄) -alkyle, S(O)₀₋₂(C₁-C₆)-alkyle, NH₂, NH-SO₂- (C₁-C₄) -alkyle, O-CH₂-COOH, O-CH₂-CO-O(C₁-C₈)-alkyle, COOH, CO-O-(C₁-C₄)-alkyle, CO-NH₂ ;
R11 signifie (C₁-C₁₂)-alkyle, (C₃-C₈)-cycloalkyle, où, dans les radicaux alkyle, un à sept atomes d'hydrogène peuvent être remplacés par fluor ; COOH, CONH₂, CO-O(C₁-C₆)-alkyle, CO-O(C₃-C₈)-cycloalkyle ; phényle, 1-naphtyle ou 2-naphtyle, biphényle, 2-pyridyle, 3-pyridyle ou 4-pyridyle, 2-furyle ou 3-furyle, 2-thiényle ou 3-thiényle ou 1-imidazolyle,
où les radicaux aryle ou hétéroaryle peuvent être jusqu'à disubstitués par F, Cl, Br, CN, OH, (C₁-C₄)-alkyle, CF₃, O-(C₁-C₄)-alkyle, S(O)₀₋₂(C₁-C₆)-alkyle, NH₂, NH-SO₂- (C₁-C₄) -alkyle, O-CH₂-COOH, O-CH₂-CO-O(C₁-C₈)-alkyle, COOH, CO-O-(C₁-C₄)-alkyle, CO-NH₂ ;
R12 signifie H, C(O)-(C₁-C₆)-alkyle ;
ainsi que leurs sels physiologiquement acceptables pour la préparation d'un médicament pour la réduction pondérale des mammifères.

2. Utilisation selon la revendication 1 pour la préparation d'un médicament destiné à la prophylaxie ou au traitement de l'obésité.

3. Utilisation selon la revendication 1 en combinaison avec une ou plusieurs substances actives réduisant le poids pour la préparation d'un médicament destiné à la réduction pondérale de mammifères.

4. Utilisation selon la revendication 1 en combinaison avec une ou plusieurs substances actives réduisant le poids pour la préparation d'un médicament destiné à la prophylaxie ou au traitement de l'obésité.

5. Utilisation selon la revendication 1 en combinaison avec la cathine, la phénylpropanolamine, l'amfépramone, le méfénorex, 1'éphédrine, la leptine, la dexamphétamine, l'amphétamine, les fenfluramine, la dexfenfluramine, la sibutramine, l'orlistat, le mazindol ou la phentermine ou leurs sels pour la préparation d'un médicament destiné à la réduction pondérale de mammifères.

6. Utilisation selon la revendication 1 en combinaison avec la cathine, la phénylpropanolamine, l'amfépramone, le méfénorex, l'éphédrine, la leptine, la dexamphétamine, l'amphétamine, la fenfluramine, la dexfenfluramine, la sibutramine, l'orlistat, le mazindol ou la phentermine ou leurs sels pour la préparation d'un médicament destiné à la prophylaxie ou au traitement de l'obésité.
